# EUROPÄISCHE PATENTSCHRIFT

(11) **EP 0 974 368 B1**
(45) Veröffentlichungstag und Bekanntmachung des Hinweises auf die Patenterteilung: **07.04.2004**
(21) Anmeldenummer: 99113464.4
(22) Anmeldetag: 13.07.1999
(51) Int. Cl.: A61L 12/12, A01N 37/16, A01N 25/30

(54) **Stabile Wirkstoffkombinationen zur Desinfektion und Reinigung von Kontaktlinsen**
Stable combinations of active agents for disinfecting and cleaning contact lenses
Combinaisons d'agents actifs pour la désinfection et le nettoyage de lentilles de contact

(30) Priorität: 23.07.1998 DE 19833173; 04.08.1998 DE 19835064
(43) Veröffentlichungstag der Anmeldung: 26.01.2000
(73) Patentinhaber: Bode Chemie GmbH & Co., 22525 Hamburg (DE)
(72) Erfinder: Kramer, Axel, Prof. Dr. med., 17487 Greifswald (DE); Rudolph, Peter, Dr., 17493 Greifswald (DE); Meyer, Brigitte, 22309 Greifswald (DE); Pietsch, Hanns, Dr., 20148 Hamburg (DE)
(74) Vertreter: Dinné, Erlend

(56) Entgegenhaltungen:
- WO-A-87/01562
- WO-A-96/28147
- GB-A- 2 178 189

## Beschreibung

Die vorliegende Erfindung betrifft eine Wirkstoffkombination auf der Basis von Magnesiummonoperphthaiat-Hexahydrat und Tensiden zur Desinfektion und Reinigung von Kontaktlinsen sowie eine Verpackung und ein Verfahren zur Herstellung derselben.

Kontaktlinsendesinfektionsmittel sind Medizinprodukte, die wegen ihres Risikopotentials in die Risikoklasse 2b eingestuft sind. An die Hygiene von Kontaktlinsen und damit auch die Leistung von Desinfektions- bzw. Reinigungsmittel für Kontaktlinsen werden dementsprechend hohe Anforderungen gestellt. Diese betreffen insbesondere das Reinigungsvermögen sowie die mikrobiologische Leistung, aber auch die Schleimhautverträglichkeit der verwendeten Mittel. Zwar kennt der Stand der Technik eine ganze Reihe von Desinfektions- bzw. Reinigungsmitteln für Kontaktlinsen, allerdings lassen die meisten der im Handel befindlichen Mittel im Hinblick auf das Desinfektionsvermögen zu wünschen übrig.

Aufgaben der vorliegenden Erfindung waren daher, die Nachteile des Standes der Technik zu vermeiden und Desinfektionsmittel zu entwickeln, welche in fester Form (wie beispielsweise als Pulver, Granulat und dergleichen mehr) ein bis drei Jahre und in wäßriger Lösung mindestens 8 bis 12 Stunden (d. h. über Nacht) stabil und dementsprechend wirksam sind.

Überraschenderweise werden diese Aufgaben gelöst durch Kontaktlinsendesinfektionsmittel mit einem Gehalt an
(a) Magnesiummonoperphthalat und
(b) Natriumcumolsulfonat,
(c) Natriumalkylbenzolsulfonat,
(d) Isotridecanolethoxylat und
(e) Methylhydroxyethylcellulose.

Zwar sind sowohl die mikrobizide Wirksamkeit als auch die gute Haut-, Schleimhautund Wundverträglichkeit sowie eine hohe Materialverträglichkeit von Monoperphthalaten und insbesondere von Magnesiummonoperphthalat bekannt, auch kennt der Stand der Technik bereits Mittel zur Desinfektion von Kontaktlinsen mit einem Gehalt an Erdalkalisalzen der Monoperphthalsäure. Allerdings konnte der Stand der Technik dennoch nicht den Weg zur vorliegenden Erfindung weisen.

Die internationale Patentanmeldung WO 87/01562 beschreibt zwar Zubereitungen zur Desinfektion von Kontaktlinsen mit einem Gehalt an Erdalkalisalzen der Monoperphthalsäure. Diese Zubereitungen enthalten gleichzeitig Komplexbildner, wie Natrium-EDTA oder Alkalisalze des Hexametaphosphates. In einer besonderen Ausführungsform liegen diese Zubereitungen auch in Form eines Granulates vor, wobei die Wirkstoffe mit Bindemitteln wie Ethylcellulose, Polyglycol oder Polyvinylpyrrolidon ummantelt sind. Allerdings ist dieser Schrift gleichzeitig zu entnehmen (vergl. Beispiel 1), daß wäßrige Lösungen des Wirkstoffes weniger als eine Stunde stabil sind und somit nicht den Anforderungen genügen, welche nach dem oben gesagten an ein Kontaktlinsendesinfektionsmittel gestellt werden.

Die Europäische Patentschrift EP 0 096 525 offenbart Desinfektionsmittel-Zusammensetzungen enthaltend eine Persauerstoff-Verbindung, ein Alkalimetallchlorid, oberflächenaktive Substanzen und Builder für Detergenzien. Diese Zubereitungen sind allerdings für eine Anwendung am menschlichen Auge und daher auch im Bereich der Kontaktlinsenpflege nicht geeignet und sind dementsprechend patentgemäß für die Desinfektion von Gegenständen, insbesondere aber von Babywindeln vorgesehen.

Die erfindungsgemäßen Kontaktlinsendesinfektionsmittel sind in jeglicher Hinsicht überaus befriedigende Präparate, die erstaunlicherweise gegenüber dem Stand der Technik den Vorteil bieten, daß sie in wäßriger Lösung mehr als 12 Stunden wirksam sind. Sie zeichnen sich darüber hinaus durch eine hervorragende Haut-, Schleimhaut- und Wundverträglichkeit sowie eine hohe Materialverträglichkeit aus.

Erfindungungsgemäß bevorzugt sind Kontaktlinsendesinfektionsmittel, welche sich durch die folgende Zusammensetzung auszeichnen:
(a) 70 bis 90 Gew.-% Magnesiummonoperphthalat,
(b) 5 bis 15 Gew.-% Natriumcumolsulfonat,
(c) 3 bis 10 Gew.-% Natriumalkylbenzolsulfonat,
(d) 0,5 bis 5,0 Gew.-% Isotridecanolethoxylat 9 EO und
(e) 0,1 bis 2,0 Gew.-% Methylhydroxyethylcellulose,
jeweils bezogen sind auf das Gesamtgewicht des Endproduktes.

Gegenstand der vorliegenden Erfindung ist auch ein Verfahren zur Herstellung von festen Wirkstoffkonzentraten, das dadurch gekennzeichnet ist, daß aus Natriumcumolsulfonat, Methylhydroxyethylcellulose und Wasser eine Sprühlösung hergestellt wird, die in einem Wirbelschichttrockner bei 60 bis 80 °C Zulufttemperatur auf eine Mischung aus Magnesiumperphthalatpulver und Natriumalkylbenzoatpulver aufgesprüht wird, und daß dieses Granulat mit einer Schmelze aus Isotridecanolethoxylat überzogen wird.

Es ist erfindungsgemäß insbesondere vorteilhaft, zur Herstellung von festen Wirkstoffkonzentraten, z. B. in (Brause-) Tabletten-, Pulver-, Pellet- oder Granulatform, 70 bis 90 Gew.-% Magnesiummonoperphthalat-Hexahydrat in einem Wirbelschichttrockner mit einer wäßrigen Lösung aus 0,1 bis 2,0 Gew.-% Methylhydroxyethylcellulose und 5 bis 15 Gew.-% Natriumcumolsulfonat bei 60 bis 80 °C Zulufttemperatur zu besprühen, zu dem Granulat anschließend 3 bis 10 Gew.-% Natriumalkylbenzolsulfonat zu mischen und diese Mischung, weiterhin im Wirbelschichttrockner, mit einer Schmelze aus 0,5 bis 5,0 Gew.-% Isotridecanolethoxylat-9 EO zu überziehen, wobei die Prozentangaben jeweils bezogen sind auf das Gesamtgewicht des Endproduktes.

Durch Sieben wird z. B. eine Korngröße von 50 bis 500 µm herausgenommen. Dieses Granulat wird beispielsweise in Portionen von 10 bis 500 mg, vorzugsweise in Portionen von 50 bis 200 mg entweder in Blisterpackungen oder Gelatinekapseln verpackt, so daß eine Gebrauchslösung jeweils frisch angesetzt werden kann. Bei Raumtemperatur hat das Granulat eine Haltbarkeit von 3 Jahren. Zur Anwendung wird jeweils eine Portion in Wasser gelöst, so daß eine 0,25 bis 1,0 Gew.-%-ige wäßrige Lösung des Wirkstoffgranulates entsteht, bezogen auf das Gesamtgewicht dieser Lösung. Die Gebrauchslösung ist ca. 24 Stunden haltbar.

### Beispiele

### Herstellung eines stabilen Granulates

Aus 19,4 Gewichtsteilen Natrium-Cumolsulfonat, 2,56 Gewichtsteilen Methylhydroxyethylcellulose und 119,6 Gewichtsteilen Wasser wird eine Lösung hergestellt, die mit Natronlauge auf einen pH-Wert von 6,5 bis 7,5 gebracht wird. Diese Lösung wird in einem Wirbelschichttrockner bei 60 bis 80°C Zulufttemperatur auf 160 Gewichtsteile Magnesiummonoperphthalat-Hexahydrat aufgesprüht. Zu diesem Granulat werden 14,8 Gewichtsteile Natriumalkylbenzolsulfonat gemischt und diese Mischung wird, weiterhin im Wirbelschichttrockner, mit einer Schmelze aus 3,2 Gewichtsteilen Isotridecanolethoxylat 9 EO überzogen. Die Komgröße wird durch die Luftgeschwindigkeit eingestellt. Ober- und Unterkom werden ausgesiebt. Das fertige Granulat hat einen Gehalt an Aktivsauerstoff von 3,9 bis 4,4 % (m/m), der pH-Wert einer 0,5 Gew.-%igen, wäßrigen Lösung beträgt 5,0 bis 5,6.

### Stabilität des Granulats

Das wie vorstehend beschrieben hergestellte Granulat wurde zu Portionen von 50 mg in Hart-PVC Blisterfolie eingefüllt und mit einer Verbundfolie aus Polyethylen und Aluminium eingesiegelt. Das derart verpackte Granulat wurde bei Raumtemperatur (22 °C) gelagert und der Aktivsauerstoffgehalt gemessen:
Ergebnisse: Sofortwert: 4,09 %, nach 24 Monaten 3,99 %, nach 36 Monaten 3,96 %

In einem weiteren Versuch wurde das gleiche Granulat zu je 50 mg in Gelatinekapseln verpackt, bei 31 °C und 40 % relativer Luftfeuchtigkeit gelagert und der Aktivsauerstoffgehalt gemessen:
Ergebnisse: Sofortwert: 4,16 %, nach 5 Monaten 3,87 %, nach 24 Monaten 3,81 % und nach 36 Monaten 3,84 %.

### Stabilität einer 0,5 Gew. % igen, wäßrigen Lösung

Das vorstehend beschriebene Granulat auf der Basis von Magnesiummonoperphthalat-Hexahydrat wurde mit Leitungswasser zu einer 0,5 Gew.-%igen, wäßrigen Lösung verdünnt und von dieser "Gebrauchslösung" wurde der Aktivsauerstoffgehalt bestimmt.
Ergebnisse: Anfangswert 0,0021 %, nach 8 Stunden bei 20 °C 0,0020 %, nach 12 Stunden bei 20 °C 0,0022 %.

### Mikrobizide Wirksamkeit einer 0,5 Gew. %igen, wäßrigen Lösung

Eine 0,5 Gew.-%ige, wäßrige Lösung des vorstehend beschriebenen Granulates auf Basis von Magnesiummonoperphthalat-Hexahydrat wurde im quantitativen Suspensionstest der deutschen Gesellschaft für Hygiene und Mikrobiologie (DGHM) auf ihre mikrobizide Wirksamkeit getestet. Als Testkeime wurden genommen: Staphylococcus aureus ATCC 6538, Pseudomonas aeruginosa ATCC 15442 sowie Candida albicans ATCC 10231, die Belastung erfolgte mit 0,2 % Albumin, die Inaktivierung durch TLHTh (Tween, Lecithin, Histidin, Thiosulfat)-Enthemmer.
Ergebnisse: In allen Fällen betrug die Keimreduktion bei 21 °C mehr als 4 logarithmische Stufen entsprechend mehr als 99,99 %.

### Schleimhautverträglichkeit einer 0,5 Gew.-%igen, wäßrigen Lösung

Die Schleimhautverträglichkeit wurde am bebrüteten Hühnerei im HET-CAM-Test geprüft. Eine 0,5 Gew.-%ige, wäßrige Lösung des vorstehend beschriebenen Granulates auf Basis Magnesiummonoperphthalat wird auf das Chorion gebracht und sodann wird die Veränderung des Gefäßsystems bewertet.
Ergebnisse: nach 30 sec 0,1a, 1a, 1a, 1a; nach 120 sec 1a, 1a, 1a, 1a,1a, 1b; nach 300 sec 1a, 1a, 1a, 1a, 1b, 1c.
Bewertung: 0= keine Reaktion, 1a= minimale Hyperämie, 1b= mäßige Hyperämie 1c= deutliche Hyperämie.

### Materialverträglichkeit einer 0,5 Gew.-% igen, wäßrigen Lösung

Die Materialverträglichkeit wurde sowohl an harten Kontaktlinsen auf der Basis von Silikonkautschuk als auch an weichen Kontaktlinsen auf der Basis von Potyhydroxyethylmethacrylat getestet. Je 12 harte und 12 weiche Kontaktlinsen wurden in eine 0,5 Gew.-%igen, wäßrigen Lösung des vorstehend beschriebenen Granulates eingelegt und dort 100 Stunden bei 22°C gelagert. Dabei wurde die Lösung alle 10 Stunden erneuert. Bewertet wurden Trübung bzw. Klarheit sowie die Dimensionsstabilität.
Ergebnis: In allen Fällen keine Trübung und keine Veränderung von Durchmesser und Dicke.

## Patentansprüche

1. Kontaktlinsendesinfektionsmittel mit einem Gehalt an
(a) Magnesiummonoperphthalat und
(b) Natriumcumolsulfonat,
(c) Natriumalkylbenzolsulfonat,
(d) Isotridecanolethoxylat und
(e) Methylhydroxyethylcellulose.

2. Kontaktlinsendesinfektionsmittel nach Anspruch 1, **dadurch gekennzeichnet, daß** es, bezogen auf das Gesamtgewicht des Endproduktes,
(a) 70 bis 90 Gew.-% Magnesiummonoperphthalat,
(b) 5 bis 15 Gew.-% Natriumcumolsulfonat,
(c) 3 bis 10 Gew.-% Natriumalkylbenzolsulfonat,
(d) 0,5 bis 5,0 Gew.-% Isotridecanolethoxylat 9 EO und
(e) 0,1 bis 2,0 Gew.-% Methylhydroxyethylcellulose
enthält.

3. Kontaktlinsendesinfektionsmittel nach einem der vorhergehenden Ansprüche, **dadurch gekennzeichnet, daß** es in Form eines Granulats vorliegt, insbesondere in Form eines Granulats, welches eine Korngröße von 50 bis 500 µm aufweist.

4. Kontaktlinsendesinfektionsmittel nach einem der vorhergehenden Ansprüche **dadurch gekennzeichnet, daß** es im Wirbelschichtverfahren erhältlich ist.

5. Kontaktlinsendesinfektionsmittel nach einem der vorhergehenden Ansprüche, **dadurch gekennzeichnet, daß** es in Portionen von 10 bis 200 mg, vorzugsweise von 40 bis 80 mg in Blisterpackungen oder in Kapseln verpackt ist.

6. Verfahren zur Herstellung von festen Wirkstoffkonzentraten, **dadurch gekennzeichnet, daß** aus Natriumcumolsulfonat, Methylhydroxyethylcellulose und Wasser eine Sprühlösung hergestellt wird, die in einem Wirbelschichttrockner bei 60 bis 80 °C Zulufttemperatur auf eine Mischung aus Magnesiumperphthalatpulver und Natriumalkylbenzoatpulver aufgesprüht wird, und daß dieses Granulat mit einer Schmelze aus Isotridecanolethoxylat überzogen wird.

7. Verfahren zur Herstellung von festen Wirkstoffkonzentraten, z. B. in (Brause-) Tabletten-, Pulver-, Pellet- oder Granulatform, **dadurch gekennzeichnet, daß** 70 bis 90 Gew.-% Magnesiummonoperphthalat-Hexahydrat in einem Wirbelschichttrockner mit einer wäßrigen Lösung aus 0,1 bis 2,0 Gew.-% Methylhydroxyethylcellulose und 5 bis 15 Gew.-% Natriumcumolsulfonat bei 60 bis 80 °C Zulufttemperatur besprüht, zu dem Granulat anschließend 3 bis 10 Gew.-% Natriumalkylbenzolsulfonat gemischt werden und diese Mischung, weiterhin im Wirbelschichttrockner, mit einer Schmelze aus 0,5 bis 5,0 Gew.-% Isotridecanolethoxylat-9 EO überzogen wird, wobei die Prozentangaben jeweils bezogen sind auf das Gesamtgewicht des Endproduktes.

## Claims

1. Contact-lens disinfectant containing
(a) magnesium monoperphthalate and
(b) sodium cumenesulphonate,
(c) sodium alkylbenzenesulphonate,
(d) isotridecanol ethoxylate and
(e) methylhydroxyethylcellulose.

2. Contact-lens disinfectant according to Claim 1, **characterized in that** it contains, based on the total weight of the final product,
(a) from 70 to 90% by weight of magnesium monoperphthalate,
(b) from 5 to 15% by weight of sodium cumenesulphonate,
(c) from 3 to 10% by weight of sodium alkylbenzenesulphonate,
(d) from 0.5 to 5.0% by weight of isotridecanol ethoxylate 9 EO, and
(e) from 0.1 to 2.0% by weight of methylhydroxyethylcellulose.

3. Contact-lens disinfectant according to one of the preceding claims, **characterized in that** it is in the form of granules, in particular in the form of granules having a particle size of from 50 to 500 µm.

4. Contact-lens disinfectant according to one of the preceding claims, **characterized in that** it is obtainable by the fluidized-bed process.

5. Contact-lens disinfectant according to one of the preceding claims, **characterized in that** it is packed in portions of from 10 to 200 mg, preferably from 40 to 80 mg, in blister packs or in capsules.

6. Process for the preparation of solid active-ingredient concentrates, **characterized in that** a spray solution is prepared from sodium cumenesulphonate, methylhydroxyethylcellulose and water and is sprayed onto a mixture of magnesium perphthalate powder and sodium alkylbenzoate powder in a fluidized-bed drier at an air intake temperature of from 60 to 80°C, and **in that** these granules are coated with a melt of isotridecanol ethoxylate.

7. Process for the preparation of solid active-ingredient concentrates, for example in (effervescent) tablet, powder, pellet or granule form, **characterized in that** from 70 to 90% by weight of magnesium monoperphthalate hexahydrate is sprayed with an aqueous solution of from 0.1 to 2.0% by weight of methylhydroxyethylcellulose and from 5 to 15% by weight of sodium cumenesulphonate in a fluidized-bed drier at an air intake temperature of from 60 to 80°C, from 3 to 10% by weight of sodium alkylbenzenesulphonate are subsequently mixed with the granules, and this mixture, still in the fluidized-bed drier, is coated with a melt of from 0.5 to 5.0% by weight of isotridecanol ethoxylate 9 EO, the percentages in each case being based on the total weight of the final product.

## Revendications

1. Agent de désinfection de lentilles de contact, qui contient:
(a) du monoperphtalate de magnésium,
(b) du cumène sulfonate de sodium,
(c) de l'alkylbenzène sulfonate de sodium,
(d) de l'éthoxylate d'isotridécanol et
(e) de la méthylhydroxyéthylcellulose.

2. Agent de désinfection de lentilles de contact selon la revendication 1, **caractérisé en ce qu'**il contient, chaque fois par rapport au poids total du produit final:
(a) de 70 à 90% en poids de monoperphtalate de magnésium,
(b) de 5 à 15% en poids de cumène sulfonate de sodium,
(c) de 3 à 10% en poids d'alkylbenzène sulfonate de sodium,
(d) de 0,5 à 5,0% en poids d'éthoxylate d'isotridécanol 9 EO et
(e) de 0,1 à 2,0% en poids de méthylhydroxyéthylcellulose.

3. Agent de désinfection de lentilles de contact selon l'une quelconque des revendications précédentes, **caractérisé en ce qu'**il se présente sous la forme d'un granulé et en particulier sous la forme d'un granulé en grains d'une taille de 50 à 500 µm.

4. Agent de désinfection de lentilles de contact selon l'une quelconque des revendications précédentes, **caractérisé en ce qu'**il est obtenu par un traitement en lit fluidisé.

5. Agent de désinfection de lentilles de contact selon l'une quelconque des revendications précédentes, **caractérisé en ce qu'**il est emballé en portions de 10 à 200 mg et de préférence de 40 à 80 mg dans des plaquettes thermoformées ou dans des gélules.

6. Procédé de préparation de concentrés solides de produits actifs, **caractérisé en ce qu'**à partir de cumène sulfonate de sodium, de méthylhydroxyéthylcellulose et d'eau, on prépare une solution de pulvérisation qui est pulvérisée dans un séchoir à lit fluidisé, à une température d'air d'amenée de 60 à 80°C, sur un mélange de poudre de perphtalate de magnésium et de poudre d'alkylbenzoate de sodium, et **en ce que** ce granulé est recouvert d'éthoxylate d'isotridécanol à l'état fondu.

7. Procédé de préparation de concentrés de produits actifs solides, par exemple sous la forme de comprimés, de poudres, de pastilles ou de granulés (à pulvériser), **caractérisé en ce que** dans un séchoir à lit fluidisé, on pulvérise une solution aqueuse constituée de 0,1 à 2,0% en poids de méthylhydroxyéthylcellulose et de 5 à 15% en poids de cumène sulfonate de sodium à une température d'air d'amenée de 60 à 80°C sur 70 à 90% en poids de monoperphtalate de magnésium hexahydraté, on mélange ensuite avec le granulé de 3 à 10% en poids d'alkylbenzène sulfonate de sodium et on recouvre ce mélange, toujours dans le séchoir à lit fluidisé, de 0,5 à 5,0% en poids d'éthoxylate d'isotridécanol 9 EO à l'état fondu, les données de pourcentage étant toutes calculées par rapport au poids total du produit final.
